# EUROPEAN PATENT APPLICATION

(11) **EP 4 635 970 A1**
(43) Date of publication of application: **22.10.2025**
(21) Application number: 23903368.1
(22) Date of filing: 06.12.2023
(51) Int. Cl.: C07K 4/00, C07K 19/00, C12N 15/87

(54) **SYNTHETIC PEPTIDE AND CONSTRUCT**

(30) Priority: 16.12.2022 JP 2022201098
(71) Applicant: Toagosei Co., Ltd., Minato-ku Tokyo 105-8419 (JP)
(72) Inventor: BAILEYKOBAYASHI, Nahoko, Tokyo 105-8419 (JP); YOSHIDA, Tetsuhiko, Tokyo 105-8419 (JP)
(74) Representative: Bals & Vogel Patentanwälte PartGmbB
(86) International application number: PCT/JP2023/043551
(87) International publication number: WO 2024/128079

(57) **Abstract**

A novel synthetic peptide with cell membrane permeability is provided. The synthetic peptide disclosed herein includes any of the following amino acid sequences: (1) an amino acid sequence in which two or more minimum constituent units are bonded continuously in series, the minimum constituent unit being GD (glycine residue-aspartic acid residue) or GE (glycine residue-glutamic acid residue); and (2) an amino acid sequence in which one to three glycine residues are bonded to a C-terminus of (1) the amino acid sequence.

## Description

### [Technical Field]

The present invention relates to a synthetic peptide with cell membrane permeability and a construct including the synthetic peptide. The present application claims priority on the basis of Japanese Patent Application No. 2022-201098 filed on December 16, 2022, and the entire content of this application is incorporated into the description of the present application by reference.

### [Background Art]

Cell penetrating peptides (CPP) are peptides that can transit from outside of a cell into at least a cytoplasm through a cell membrane. Japanese Patent No. 7041853 discloses a carrier peptide fragment that functions as the CPP and a technique of transferring a target foreign substance into a eukaryotic cell by using the carrier peptide fragment.

### [Citation List]

### [Patent Literature]

[Patent Literature 1] Japanese Patent No. 7041853

### [Summary of Invention]

### [Technical Problem]

Incidentally, an amino acid sequence constituting the CPP generally includes a basic amino acid (for example, arginine or lysine), and it has been said that the basic amino acid can contribute to the efficiency of cell membrane permeability. It has been known that, however, when the ratio of the basic amino acid in the CPP is high, for example, the cell toxicity generally tends to become high.

In view of this, it is a main object of the present invention to provide a novel synthetic peptide with cell membrane permeability that does not include a basic amino acid. It is another object of the present invention to provide a construct including the synthetic peptide.

### [Solution to Problem]

A synthetic peptide disclosed herein is a CPP capable of transferring a target foreign substance from outside a eukaryotic cell into at least a cytoplasm of the cell. The synthetic peptide includes any of the following amino acid sequences:
(1) an amino acid sequence in which two or more minimum constituent units are bonded continuously in series, the minimum constituent unit being GD (glycine residue-aspartic acid residue) or GE (glycine residue-glutamic acid residue); and
(2) an amino acid sequence in which one to three glycine residues are bonded to a C-terminus of (1) the amino acid sequence.

The synthetic peptide with the aforementioned structure does not include an arginine residue, a lysine residue, or a histidine residue known as a basic amino acid. As described above, it has been considered that the conventional amino acid residue can exhibit the high cell membrane permeability because of having the basic amino acid; however, the synthetic peptide disclosed herein can exhibit the high cell membrane permeability despite not having the basic amino acid.

In one aspect of the synthetic peptide disclosed herein, each of (1) the amino acid sequence and (2) the amino acid sequence includes two or more and six or less of the minimum constituent units. That is to say, the synthetic peptide disclosed herein can include the amino acid sequence in which the minimum constituent unit is repeated twice or more and six times or less.

The synthetic peptide disclosed herein includes, for example, any of the following amino acid sequences:
GDGD (SEQ ID No.: 1);
GEGE (SEQ ID No.: 2);
GDGEG (SEQ ID No.: 7);
GEGDG (SEQ ID No.: 8);
GDGDGDG (SEQ ID No.: 9);
GDGDGDGDG (SEQ ID No.: 10);
GDGDGDGDGDG (SEQ ID No.: 11);
GDGDGDGDGDGDG (SEQ ID No.: 12);
GEGEGEGEGEGEG (SEQ ID No.: 13); and
GDGEGDGEGDGEG (SEQ ID No.: 14).
In any of the amino acid sequences described above, the high cell membrane permeability can be exhibited.

In addition, in order to achieve the aforementioned object, the present disclosure provides a construct for transferring a foreign substance (hereinafter also referred to as "construct" simply) that is manufactured in order to transfer a target foreign substance from outside the eukaryotic cell into at least the cytoplasm of the cell. The construct disclosed herein includes the synthetic peptide disclosed herein and the target foreign substance that is bonded to an N-terminus and/or a C-terminus of the synthetic peptide. Since this construct includes the synthetic peptide that functions as the CPP, the construct is transferred efficiently into the eukaryotic cell and the target foreign substance can be transferred efficiently into the cell.

In one aspect of the construct disclosed herein, the foreign substance can be at least one kind of organic compound selected from the group consisting of a polypeptide, a nucleic acid, a dye, and a drug.

Here, the term "polypeptide" refers to a polymer with a structure in which a plurality of amino acids are linked by peptide bonds. The polypeptide is not limited by the number of peptide bonds (that is, the number of amino acid residues). In other words, the term "polypeptide" encompasses those generally called peptides with about 10 or more and less than 300 amino acid residues and those generally called proteins (typically, high molecular compounds with 300 or more amino acid residues). In this field, polypeptides and proteins are not distinguished strictly. In this specification, polymers formed of a plurality of amino acid residues (including oligomers) are collectively referred to as polypeptides.

Moreover, the term "nucleic acid" refers to a nucleotide polymer and includes DNA and RNA. The term "nucleic acid" is not limited by the number of bases.

In one aspect of the construct disclosed herein, the foreign substance can be disposed on the C-terminus of the synthetic peptide.

### [Brief Description of Drawings]

Fig. 1 is a graph showing values of MFI obtained by culturing with constructs (additives) shown in Examples 1 to 9 and Reference Example 1 added to culture solutions of NSC-34 cells, and then analyzing the cells with a flow cytometer.
Fig. 2 is a graph showing values of MFI obtained by culturing with constructs (additives) shown in Examples 10 to 12 and Reference Example 2 added to culture solutions of NSC-34 cells, and then analyzing the cells with the flow cytometer.

### [Description of Embodiments]

Some embodiments of the art disclosed herein will be described below. Matters that are other than matters particularly mentioned in the present specification and that are necessary for the implementation of the present art (for example, general matters related to chemical synthesis methods for peptide, cell culture techniques, and preparation of constructs containing peptides or nucleic acids as a component) can be grasped as design matters of those skilled in the art based on the prior art in the fields such as cell engineering, physiology, medical science, pharmacology, organic chemistry, biochemistry, genetic engineering, protein technology, molecular biology, and genetics. Moreover, the art disclosed herein can be carried out on the basis of the contents disclosed in this specification and common technical knowledge in the fields. In the following description, the amino acids are represented by single letter codes based on the nomenclature for amino acids in the IUPAC-IUB guidelines in some cases. Note that the term "amino acid residue" in this specification encompasses N-terminus amino acids and C-terminus amino acids of a peptide chain unless otherwise stated specifically.

In this specification, the term "synthetic peptide" refers to a peptide fragment whose peptide chain alone does not stably exist in nature. The synthetic peptide is manufactured by artificial chemical synthesis or biosynthesis (that is, production based on genetic engineering) and can stably exist in a predetermined composition. Here, the term "peptide" refers to an amino acid polymer having a peptide bond (including a dimer, a trimer, an oligomer, or the like) and is not limited by the number of amino acid residues.

The amino acid residue of the peptide or protein in this specification may be either an L-form or a D-form. Note that the amino acid sequence in this specification always represents the N-terminus on the left side and the C-terminus on the right side.

In one aspect of the synthetic peptide disclosed herein, the synthetic peptide includes the following (1) or (2) amino acid sequence:
(1) an amino acid sequence in which two or more minimum constituent units are bonded continuously in series, the minimum constituent unit being GD (glycine residue-aspartic acid residue) or GE (glycine residue-glutamic acid residue); and
(2) an amino acid sequence in which one to three glycine residues are bonded to a C-terminus of (1) the amino acid sequence.

In the amino acid sequence constituting the synthetic polypeptide, two or more of only one of GD and GE, which are the minimum constituent units, may be bonded continuously in series. Alternatively, two or more of both GD and GE, which are the minimum constituent units, may be mixed and bonded continuously in series. For example, GD and GE may be bonded continuously in series so as to be disposed alternately. Since both an aspartic acid and a glutamic acid are acidic amino acids, the synthetic peptide disclosed herein can be said to have a repeated sequence in which a glycine residue and an acidic amino acid residue are repeated. In addition, "bonded continuously in series" means that, to the N-terminus and/or the C-terminus of one minimum constituent unit, the other minimum constituent unit is bonded through the peptide bond.

In the synthetic peptide disclosed herein, for example, two or more and ten or less of the minimum constituent units may be bonded continuously in series. Alternatively, two or more and six or less, three or more and six or less, four or more and six or less, or five or more and six or less of the minimum constituent units may be bonded continuously in series. In a preferred aspect, six of the minimum constituent units are bonded continuously in series. The amino acid sequence in which six of the minimum constituent units are bonded continuously in series can exhibit the particularly excellent cell membrane permeability.

As indicated in (2) above, in the synthetic peptide disclosed herein, one to three glycine residues may be bonded to the C-terminus of the amino acid sequence in which the minimum constituent units are bonded continuously in series, and the number of glycine residues is preferably one or two, and more preferably one. The glycine residue is the residue with the smallest side chain among the amino acids, and is a neutral amino acid. Therefore, it can be understood from the technical common sense in the present technical field that, in the case where the amino acid sequence in which the minimum constituent units are bonded continuously in series has the cell membrane permeability, even if one to three glycine residues are bonded to the C-terminus of the amino acid sequence, the cell membrane permeability will not be deteriorated remarkably.

The number of amino acid residues of the synthetic peptide disclosed herein is four or more, five or more, six or more, seven or more, eight or more, nine or more, ten or more, eleven or more, or twelve or more because at least two of the minimum constituent units are included. The upper limit of the number of amino acid residues of the synthetic peptide is not limited in particular and can be, for example, 21 or less, 20 or less, 19 or less, 18 or less, 17 or less, 16 or less, 15 or less, 14 or less, or 13 or less. In a case where the synthetic peptide includes too many amino acid residues, the synthetic peptide becomes too bulky, which may result in the decrease in cell membrane permeability.

Specific examples of the amino acid sequence of the synthetic peptide disclosed herein that includes two of the minimum constituent units include GDGD (SEQ ID No.: 1), GEGE (SEQ ID No.: 2), GDGE (SEQ ID No.: 3), GEGD (SEQ ID No.: 4), GDGDG (SEQ ID No.: 5), GEGEG (SEQ ID No.: 6), GDGEG (SEQ ID No.: 7), and GEGDG (SEQ ID No.: 8).

Specific examples of the amino acid sequence of the synthetic peptide disclosed herein that includes three or more of the minimum constituent units include GDGDGDG (SEQ ID No.: 9), GDGDGDGDG (SEQ ID No.: 10), GDGDGDGDGDG (SEQ ID No.: 11), GDGDGDGDGDGDG (SEQ ID No.: 12), GEGEGEGEGEGEG (SEQ ID No.: 13), and GDGEGDGEGDGEG (SEQ ID No.: 14).

Note that the synthetic peptide disclosed herein can be a modified sequence of the amino acid sequence shown in (1) or (2) described above unless the cell membrane permeability is deteriorated remarkably. Here, the "modified sequence" corresponds to an amino acid sequence (modified amino acid sequence) formed by the substitution, deletion, and/or addition (insertion) of one or several (typically, two or three) amino acid residues.

Typical examples of the modified sequence in this specification include a sequence produced by so-called conservative amino acid replacement in which one, two, or three amino acid residues are conservatively replaced, a sequence in which one, two, or three amino acid residues are added (inserted) to or deleted from a predetermined amino acid sequence, and the like. Typical examples of the conservative amino acid replacement include a sequence in which the glycine residue corresponding to a nonpolar amino acid is replaced by another nonpolar amino acid residue such as an alanine residue, and the like.

Since the aforementioned synthetic peptide disclosed herein can have the cell membrane permeability, a target foreign substance can be transferred from outside a eukaryotic cell into at least a cytoplasm (also into a nucleus) of the cell. Thus, the present disclosure provides a construct for transferring a foreign substance including the synthetic peptide disclosed herein.

The construct disclosed herein includes the aforementioned synthetic peptide disclosed herein and the target foreign substance that is bonded to the N-terminus and/or the C-terminus of the synthetic peptide.

The construct disclosed herein can be designed and configured by bonding (linking), either directly or indirectly via a suitable linker, a desired foreign substance to the N-terminus and/or the C-terminus of the abovementioned synthetic peptide.

The linker is not limited in particular and may be either a peptide linker or a non-peptide linker. Although there is no particular limitation, the amino acid sequence of the peptide linker is preferably a flexible amino acid sequence that does not cause steric hindrance. Examples of the possible peptide linker include linkers including ten or less (more preferably one or more and five or less, for example one, two, three, four, or five amino acid residues) amino acid residues including one kind or two or more kinds of amino acid residues selected from glycine, alanine, serine, and the like. As such a linker, β-alanine may be used. As the non-peptide linker, for example, an alkyl linker, a polyethylene glycol (PEG) linker, an amino hexanoyl spacer, or the like can be used, without particular limitations.

The foreign substance can be, for example, an organic compound such as a polypeptide, a nucleic acid, a dye, or a drug.

If the foreign substance is a polypeptide, the target construct for transferring a foreign substance can be manufactured in such a way that a peptide chain is designed to contain the amino acid sequence constituting the polypeptide and the amino acid sequence constituting the synthetic peptide, and then the peptide chain is subjected to biosynthesis or chemical synthesis. Moreover, the construct can be prepared in such a way that an organic compound that acts as a nucleic acid such as various types of DNA or RNA, a dye (for example, various types of fluorescent compounds such as FAM and FITC), or a drug (for example, an antitumor drug including a nucleic acid-based antitumor drug such as 5-fluorouracil (5FU), an antiviral drug such as azidothymidine (AZT), etc.) is directly or indirectly bonded to the N-terminus and/or the C-terminus of the above synthetic peptide by various publicly known scientific methods. Although there is no particular limitation, the function of the foreign substance can be, for example, promoting differentiation induction of stem cells (stem cell differentiation inducing activity), growth inhibition of tumor cells (antitumor activity), growth inhibition of virus-infected cells (antivirus activity), or the like.

In the construct disclosed herein, the number of foreign substances to be bonded to the synthetic peptide is not limited in particular. For example, one or more foreign substances may be bonded to one synthetic peptide. Although there is no particular limitation, for example, a polypeptide, a nucleic acid, a drug, or the like may be bonded to the C-terminus of one synthetic peptide and then a dye may be bonded to the N-terminus thereof. It is preferable to bond the dye to the synthetic peptide because it becomes easy to evaluate the efficiency of transferring the construct into the eukaryotic cell and the localization in the cell.

Note that when the foreign substance is a polypeptide, the polypeptide (amino acid sequence) to be used is not particularly limited. For example, a polypeptide with about 100 to 1000 amino acid residues or proteins, which have a relatively large number of amino acid residues, can also be used as the foreign substance.

Typically, the total number of amino acid residues constituting the synthetic peptide manufactured as the construct for transferring a foreign substance is several to several tens (for example, 10) or more and suitably 1000 or less, preferably 600 or less, more preferably 500 or less, and particularly preferably 300 or less (for example, 10 to 300). The polypeptide with such a length is easy to synthesize (biosynthesis, chemical synthesis) and easy to use.

Preferably, the foreign substance to be used is a mature form or precursor (including pro-forms and prepro-forms) of a polypeptide involved in a function such as the development, differentiation, growth, malignant transformation, homeostasis, or regulation of metabolism in various cells and tissues (organs). Moreover, the method for transferring a foreign substance disclosed herein can be carried out to transfer a polypeptide with a heretofore unknown function into a cell to elucidate the function of the polypeptide in the cell (in a biological tissue).

For example, if the eukaryotic cell that is the target to which the foreign substance is to be transferred is a human or other mammalian stem cell, it is preferable to use a mature form or precursor of a polypeptide with various biological activities related to the differentiation induction of the stem cells. Note that the term "stem cell" encompasses somatic stem cells, embryonic stem cells, and induced pluripotent stem cells (hereinafter, iPS cells). Moreover, when the eukaryotic cell to which the foreign substance is to be transferred is a cancer cell (tumor cell), it is preferable to use various polypeptides involved in the induction of apoptosis of the cancer cell (tumor cell). Alternatively, in this case, it is preferable to use a polypeptide that can prevent a cancer cell (tumor cell) from inhibiting the function of an immunity monitoring mechanism. Moreover, when the eukaryotic cell that is the target of transfer is a bacteria-infected cell or a virus-infected cell, it is preferable to use various polypeptides involved in inducing apoptosis of these infected cells, a polypeptide that can inhibit the increase of bacteria or virus in the infected cells, or a polypeptide that can inhibit the expansion of the bacteria or virus infection from the infected cells. Furthermore, similarly to the synthetic peptide, the polypeptide as the foreign substance may include a modified amino acid sequence that is formed by the replacement, deletion, and/or addition (insertion) of one or several amino acid residues as long as that function is kept.

In the construct in which the foreign substance is bonded to the C-terminus of the synthetic peptide, an α-amino group of the amino acid residue of the N-terminus of the synthetic peptide is preferably acetylated. Although the detailed mechanism is not clear, since the α-amino group of the amino acid of the N-terminus in most of the proteins in the eukaryotic cell is subjected to the acetylation modification, such a structure can improve the stability of the construct in the cells.

In the construct, the amino acid residue of the C-terminus is preferably amidated. The structural stability (for example, protease resistance) of the construct as described above in the cytoplasm and nucleolus can be improved by amidation of a carboxyl group of the amino acid residue (typically, a C-terminal amino acid residue of a peptide chain). In addition, the amidation of the carboxyl group improves the hydrophilicity of the construct, so that the solubility of this construct in an aqueous solvent can be improved. Examples of such an aqueous solvent include water, various buffer solutions, physiological saline (for example, PBS), a cell culture solution, and the like. For example, in the case of the construct in which the foreign substance is bonded to the N-terminus of the synthetic peptide, a carboxyl group of the amino acid residue of the C-terminus of the synthetic peptide is preferably amidated. In a case where, for example, the foreign substance is a polypeptide and this polypeptide is bonded to the C-terminus of the synthetic peptide, it is preferable to amidate the carboxyl group of the C-terminal amino acid residue of the polypeptide.

Among constructs, those with a relatively short peptide chain (encompassing a polypeptide, a synthetic peptide, and a peptide linker configured as a foreign substance) can easily be manufactured by a general chemical synthesis method. For example, either a conventionally known solid phase or liquid phase synthesis method can be used. A solid phase synthesis method using Boc (t-butyloxycarbonyl) or Fmoc (9-fluoroenylmethoxycarbonyl) as a protecting group for an amino group is preferred. In other words, the aforementioned peptide chain with the desired amino acid sequence and modifications (N-terminal acetylation, C-terminal amidation, etc.) can be synthesized by a solid phase synthesis method using a commercially available peptide synthesizer. Note that only a part of the peptide chain may be synthesized by the aforementioned method and for example, the peptide chain including only the synthetic peptide or including the synthetic peptide and the peptide linker can be synthesized.

Alternatively, the peptide part may be manufactured by biosynthesis in accordance with the genetic engineering method. In other words, a polynucleotide (typically, DNA) of a nucleotide sequence (including the ATG start codon) that codes for the desired amino acid sequence is synthesized. Then, a recombinant vector with a genetic construct for expression that includes the synthesized polynucleotide (DNA) and various regulatory elements (including a promoter, ribosome binding site, terminator, enhancer, and various cis-elements for controlling the level of expression) to express the amino acid sequence in a host cell is configured in accordance with the host cell.

Using the general techniques, this recombinant vector is transferred to a predetermined host cell (for example, yeast, insect cell, or plant cell), and the host cell, or an individual or tissue containing the cell is cultured under a predetermined condition. The target peptide can be produced in the cell thereby. Furthermore, the target peptide part can be obtained by isolating the peptide part from the host cell (or from a culture medium if it is secreted) and if necessary, refolding and purifying the peptide part, for example.

Note that the method for configuring the recombinant vector and the method for transferring the configured recombinant vector to the host cell, etc., may utilize methods conventionally used in the fields without modification, and because those methods themselves are not a characterizing feature of the present art, the detailed explanation thereof is omitted.

For example, a fusion protein expression system can be used for efficient, large volume production in host cells. More specifically, first, the gene (DNA) coding for the amino acid sequence of the target polypeptide is obtained by chemical synthesis, and the synthesized gene is transferred to a suitable site in a suitable fusion protein expression vector (for example, a glutathione S-transferase (GST) fusion protein expression vector such as the pET series provided by Novagen and the pGEX series provided by Amersham Biosciences). Then, the host cells (typically E. coli) are transformed by the vector. The resulting transformant is cultured to prepare the target fusion protein. Next, the protein is extracted and purified. Then, the resulting purified fusion protein is cleaved by a predetermined enzyme (protease) and the freed target peptide fragment (i.e., the designed artificial polypeptide) is recovered by a method such as affinity chromatography. The target construct (artificial polypeptide) can be manufactured using this kind of conventionally known fusion protein expression system (for example, the GST/His system provided by Amersham Biosciences can be utilized).

Alternatively, template DNA for use in a cell-free protein synthesis system (i.e., a synthetic gene fragment containing a nucleotide sequence coding for the amino acid sequence of the peptide part for the construct) can be configured, and in-vitro synthesis of the target polypeptide can be carried out by employing a so-called cell-free protein synthesis system using various compounds necessary for synthesis of the peptide part (ATP, RNA polymerase, amino acids, etc.). References concerning a cell-free protein synthesis system include, for example, the papers by Shimizu et al. (Shimizu et al., Nature Biotechnology, 19, 751-755 (2001)) and Madin et al. (Madin et al., Proc. Natl. Acad. Sci. USA, 97(2), 559-564 (2000)). At the time of the filing of the present application, there are already many companies carrying out polypeptide production on consignment on the basis of the technology disclosed in these documents, and cell-free protein synthesis kits are commercially available (for example, obtainable from Cell Free Sciences Co., Ltd. in Japan).

The nucleotide sequence coding for the peptide part of the construct and/or a single-chain or double-chain polynucleotide including the nucleotide sequence that is complementary to the above-described nucleotide sequence can be manufactured (synthesized) easily by the conventionally known method. That is to say, by selecting the codon that corresponds to each amino acid residue constituting the designed amino acid sequence, the nucleotide sequence corresponding to such an amino acid sequence is easily determined and provided. Once the nucleotide sequence is determined, the polynucleotide (single chain) corresponding to the desired nucleotide sequence can be easily obtained using a DNA synthesizer or the like. Furthermore, by using the obtained single-chain DNA as a template, the target double-chain DNA can be obtained with various enzymatic synthesizing means (typically, PCR). Moreover, the polynucleotide may be either in a DNA form or an RNA (mRNA, etc.) form. DNA can be provided as a double chain or a single chain. In the case of providing DNA as a single chain, the chain may be either a code chain (sense chain) or a non-code chain (anti-sense chain) with the sequence complementary to that code chain. The polynucleotide obtained in this manner can be used as a material for configuring a recombination gene (expression cassette) for peptide production in various host cells or a cell-free protein synthesis system as described above.

The construct disclosed herein can be suitably used as an effective component of the composition for the usage based on the function of the foreign substance. Note that the construct may be in a salt form unless the function of the foreign substance is lost. For example, an acid addition salt that can be obtained by carrying out an addition reaction with a normally used inorganic or organic acid in accordance with a conventional method can be used. Thus, the "construct" according to the present specification and the scope of claims can encompass the construct in such a salt form.

The construct can be used as an effective component of the composition that can contain various carriers that are allowable in terms of medical (pharmaceutic) perspectives in accordance with the usage mode. As the carrier, for example, a carrier that is generally used in the peptide medical fields such as a diluent or an excipient is preferable. As such a carrier, typically, water, a physiological buffer, and various organic solvents are given, although depending as appropriate on the usage or mode of the construct for transferring a foreign substance. Moreover, the carrier can be an alcohol (such as ethanol) aqueous solution with a suitable concentration, glycerol, or non-drying oil such as olive oil, or may be liposome. As a secondary component that can be contained in a pharmaceutical composition, various filling agents, thickening agents, bonding agents, moisture adding agents, surfactants, dyes, flavoring agents, and the like are given.

The mode of the composition is not limited in particular. For example, modes of liquids, suspensions, emulsions, aerosols, foams, granules, powders, tablets, capsules, and ointments are given. Moreover, for the use in injection or the like, a lyophilized product or granulated product to prepare a drug solution by being dissolved in physiological saline or a suitable buffer (e.g., PBS), etc., just before use can be formed.

The conventionally known methods may be used for the processes themselves to prepare various modes of medicines (compositions) from the construct (main component) and various carriers (secondary components) as materials, and a detailed explanation of such a manufacturing process itself is omitted herein because it is not a characterizing feature of the present art. For example, Comprehensive Medicinal Chemistry, edited by Corwin Hansch, Pergamon Press, 1990, can be noted as a source of detailed information concerning formulations.

By using the construct disclosed herein, the foreign substance can be transferred in vivo or in vitro. Roughly speaking, the transfer method can include: a step (preparing step) of preparing a construct disclosed herein; and a step (supplying step) of supplying the construct into a sample including a target eukaryotic cell. The method can further include, after the supplying step, a step (transferring step) of incubating the sample to which the construct has been supplied, thereby transferring the construct into the eukaryotic cell in the sample.

The term "eukaryotic cell" encompasses, in vivo, various tissues, viscera, organs, blood, lymph, and the like, for example. The term "eukaryotic cell" encompasses, in vitro, various cell clusters, tissues, viscera, organs, blood, lymph, cell lines, and the like extracted from a body, for example.

The composition including the construct disclosed herein can be used in vivo in dosage and method according to the mode and intended purpose. For example, exactly a desired amount of liquid can be administered to a diseased area (for example, malignant tumor tissue, virus-infected tissue, inflamed tissue, etc.) of a patient (i.e., the body) by intravenous, intramuscular, subdermal, intradermal, or intraperitoneal injection. Alternatively, the composition in a solid mode such as a tablet or in a gel-form or aqueous jelly-form such as ointment can be applied directly to a predetermined tissue (i.e., for example, a diseased area such as an organ or a tissue including a tumor cell, virus-infected cell, inflamed cell, or the like). Further alternatively, the composition in a solid mode such as a tablet can be administered orally. In the case of the oral administration, it is preferable to perform encapsulation or apply a protection (coating) material in order to suppress decomposition by digestive enzymes in the alimentary canal.

Alternatively, the construct in an amount suitable for the eukaryotic cell cultured in vitro is preferably supplied to a culture solution of the target eukaryotic cell at least once. The amount per supply and the number of times of supplies are not limited in particular because they can vary depending on the conditions including the kind of eukaryotic cells to culture, the cell density (cell density at the start of culture), the passage number, the culture condition, the kind of culture medium, and the like. For example, the composition is preferably added once, twice, or more times so that the concentration of the synthetic peptide in the culture solution falls within the range of about 0.05 µM or more and 100 µM or less, 0.5 µM or more and 50 µM or less, or 1 µM or more and 30 µM or less, for example. In addition, the incubating time after the construct is added is not limited in particular either because the incubating time can vary depending on the kind of eukaryotic cells and various conditions. For example, the incubating time can be 0.5 hours or more, 1 hour or more, 4 hours or more, 8 hours or more, or 20 hours or more. Moreover, the incubating condition is not limited in particular because the incubating condition can vary depending on the kind of eukaryotic cells; however, the incubation is possible in a 5% CO₂ atmosphere under 37°C, for example. Note that one example of the transferring method in vitro will be described in test examples below.

A method for evaluating the transferring efficiency of the construct is not limited in particular. For example, in the case where a dye (typically, a fluorescent compound) is bonded to the construct, it is possible to evaluate the transferring efficiency into the eukaryotic cells by using microscope observation (for example, fluorescence microscope observation), flow cytometry, or the like. Alternatively, the transferring efficiency of the construct can be evaluated by an immunochemical method (for example, Western Blot, immunocytochemistry, or the like) that uses an antibody to recognize the peptide part of the construct specifically.

As described above, the following items are given as specific aspects of the art disclosed herein.
Item 1: The synthetic peptide for transferring the target foreign substance from outside the eukaryotic cell into at least the cytoplasm of the cell, the synthetic peptide including any of the following amino acid sequences:
   (1) the amino acid sequence in which two or more of the minimum constituent units are bonded continuously in series, the minimum constituent unit being GD (glycine residue-aspartic acid residue) or GE (glycine residue-glutamic acid residue); and
   (2) the amino acid sequence in which one to three glycine residues are bonded to the C-terminus of (1) the amino acid sequence.
Item 2: The synthetic peptide according to Item 1, in which each of (1) the amino acid sequence and (2) the amino acid sequence includes two or more and six or less of the minimum constituent units.
Item 3: The synthetic peptide for transferring the target foreign substance from outside the eukaryotic cell into at least the cytoplasm of the cell, the synthetic peptide including any of the following amino acid sequences:
   GDGD (SEQ ID No.: 1);
   GEGE (SEQ ID No.: 2);
   GDGEG (SEQ ID No.: 7);
   GEGDG (SEQ ID No.: 8);
   GDGDGDG (SEQ ID No.: 9);
   GDGDGDGDG (SEQ ID No.: 10);
   GDGDGDGDGDG (SEQ ID No.: 11);
   GDGDGDGDGDGDG (SEQ ID No.: 12);
   GEGEGEGEGEGEG (SEQ ID No.: 13); and
   GDGEGDGEGDGEG (SEQ ID No.: 14).
Item 4: The construct for transferring the foreign substance that is manufactured in order to transfer the target foreign substance from outside the eukaryotic cell into at least the cytoplasm of the cell, the construct including: the synthetic peptide according to any one of Items 1 to 3; and the target foreign substance that is bonded to the N-terminus and/or the C-terminus of the synthetic peptide.
Item 5: The construct according to Item 4, in which the foreign substance is at least one kind of organic compound selected from the group consisting of a polypeptide, a nucleic acid, a dye, and a drug.
Item 6: The construct according to Item 4 or 5, in which the foreign substance is disposed on the C-terminus of the synthetic peptide.

Since Item 3 described above indicates the specific example including the matter specified by Item 1 or 2, Item 3 can be subordinate to Item 1 or 2.

Several test examples concerning the art disclosed herein are described below, but it is not intended to limit the art disclosed herein to these test examples.

### [Test 1]

### <Manufacture of construct>

Constructs including synthetic peptides with amino acid sequences shown in Table 1 were prepared. A construct including a peptide n (n is a natural number of 1 to 9) shown in Table 1 was defined as a sample n, and samples 1 to 9 were obtained from Eurofins Genomics K.K. In the prepared samples 1 to 9, the α-amino group of the amino acid residue of the N-terminus in the peptides 1 to 9 were acetylated. Next, FAM (C₂₁H₁₂O₇: 5(6)-carboxyfluorescein, molecular weight 376.3, excitation wavelength 495 nm, fluorescence wavelength 520 nm) corresponding to fluorescent dye was bonded as the foreign substance to the amino acid residue of the C-terminus of each of the peptides 1 to 9 in the preparation.

### [Table 1]

**Table 1**

| Peptide No. | Amino acid sequence | SEQ ID No. |
|---|---|---|
| 1 | GDGD | 1 |
| 2 | GEGE | 2 |
| 3 | GDGEG | 7 |
| 4 | GEGDG | 8 |
| 5 | GDGDGDG | 9 |
| 6 | GDGDGDGDG | 10 |
| 7 | GDGDGDGDGDG | 11 |
| 8 | GD | - |
| 9 | GE | - |

### <Evaluation of cell membrane permeability by flow cytometry>

As the eukaryotic cells, NSC-34 cells (mouse motor neuron-like hybrid cell line) were used and the cell membrane permeability of the peptides 1 to 9 was analyzed. As a culture medium of the NSC-34 cell, 10% fetal bovine serum (FBS)-containing Dulbecco's modified Eagle's medium (DMEM (Cat No. 044-29765, manufactured by FUJIFILM Wako Pure Chemical Corporation)) was used. The samples 1 to 9 were each dissolved in dimethyl sulfoxide (DMSO) to prepare sample solutions 1 to 9 with a sample concentration of 4 mM. Then, the sample solutions were diluted in the culture medium to prepare 40 µM sample solutions 1 to 9. The aforementioned prepared sample solutions 1 to 9 were used in Examples 1 to 9, and the FAM solution was used in Reference Example 1.

### (Example 1)

The NSC-34 cell was suspended in the culture medium to prepare a cell suspension of 2 × 10⁵ cells/mL. The cell suspension was added by 1 mL to a well of a commercially available six-hole (well) plate (manufactured by AGC TECHNO GLASS Co., Ltd.) so as to seed the NSC-34 cell to be 2 × 10⁵ cells/well. Next, 1 mL of the 40 µM sample solution 1 was added to the well so that the sample concentration in the culture medium in the well became 20 µM. After that, the six-well plate was left at rest in a cell culture apparatus, and incubated for 20 hours at 37°C under a 5% CO₂ condition.

After the 20-hour incubation, the culture supernatant was removed from the well and the cells in the well were cleaned twice with 1 mL of PBS. Next, 100 µL of the 0.25% trypsin/EDTA solution was added to the well and the incubation was performed for three minutes at 37°C. After the incubation, 900 µL of the culture medium was added to the well to deactivate the trypsin. Subsequently, the cell suspension in the well was transferred to a tube and the cells were collected. This tube was subjected to five-minute centrifugal separation at 4°C under a condition of 210 × g. After the centrifugal separation, the supernatant was removed and the precipitate (cell pellet) was suspended (cleaned) with 1 mL of PBS, which was followed by the centrifugal separation under the same condition as that described above. After this operation was repeated twice, the supernatant was removed and thus, the cells (cell pellet) cultured in the culture medium containing the sample 1 were obtained.

Regarding the obtained cells (cell pellet), the cell membrane permeability of the sample 1 was analyzed using a flow cytometer. As the flow cytometer, On-Chip Flowcytometer (manufactured by On-Chip Biotechnologies Co., LTD.) was used.

For such an analysis, the obtained cell pellet was suspended with 100 µL of on-chip T buffer to prepare a cell suspension for analysis.

Using the aforementioned flow cytometer, gating based on forward scatter (FSC) and side scatter (SSC) was performed to set a gate about a cell group to be analyzed, and the fluorescence intensity was measured about the cell group in this gate. Note that the number of cells of the cell group was set to at least 10000 or more in the analysis. The fluorescence intensity was measured using a fluorescence detector FL2 of the flow cytometer (optimum detection wavelength around 543 nm) capable of detecting the fluorescence wavelength of FAM. Regarding this measurement result, the analysis was performed using commercially available analysis software "FlowJo" (manufactured by TreeStar) to obtain mean fluorescence intensity (MFI) of the cell group to be measured.

### (Examples 2 to 9)

The process similar to that in Example 1 was performed except that the sample solution 1 was changed to any one of sample solutions 2 to 9. Note that the sample (construct) used in each Example is shown in Table 2.

### (Reference Example 1)

The process similar to that in Example 1 was performed except that the sample 1 was changed to the fluorescent dye FAM. Note that the FAM concentration in the culture medium containing the FAM was the same as the concentration of the sample 1 in Example 1 (that is, the FAM concentration was 20 µM in the culture medium in the well).

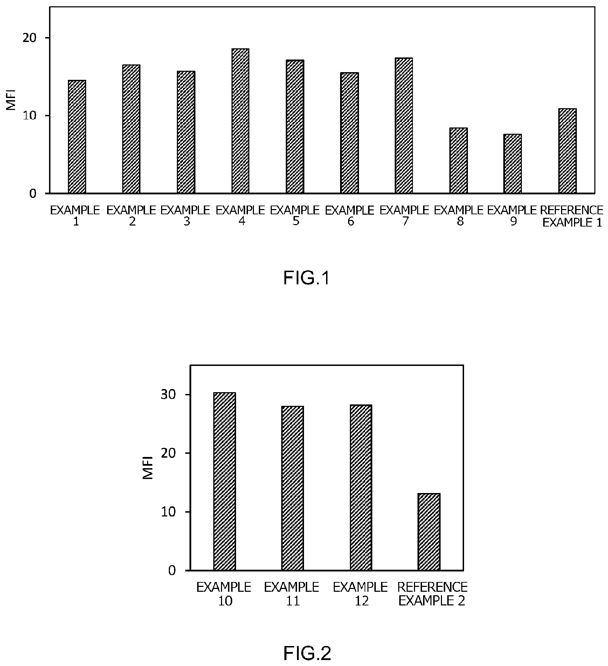

The results obtained from Examples 1 to 9 and Reference Example 1 are shown in Table 2 and Fig. 1. Fig. 1 is a graph showing values of MFI in Examples.

### [Table2]

**Table 2**

| | Structure of construct (additive) | MFI |
|---|---|---|
| Example 1 | Ac-GDGD-FAM | 14.5 |
| Example 2 | Ac-GEGE-FAM | 16.5 |
| Example 3 | Ac-GDGEG-FAM | 15.7 |
| Example 4 | Ac-GEGDG-FAM | 18.6 |
| Example 5 | Ac-GDGDGDG-FAM | 17.1 |
| Example 6 | Ac-GDGDGDGDG-FAM | 15.5 |
| Example 7 | Ac-GDGDGDGDGDG-FAM | 17.4 |
| Example 8 | Ac-GD-FAM | 8.4 |
| Example 9 | Ac-GE-FAM | 7.6 |
| Reference Example 1 | FAM | 10.9 |

As shown in Table 2 and Fig. 1, the value of MFI was higher in Examples 1 to 7 than in Reference Example 1. That is to say, the samples 1 to 7 in which any of the peptides 1 to 7 was bonded to the FAM were transferred in the cells more than in the case of adding the FAM alone (Reference Example 1). This indicates that the peptides 1 to 7 have the cell membrane permeability.

On the other hand, the value of MFI was lower in Examples 8 and 9 than in Reference Example 1. This indicates that the peptides 8 and 9 do not have the cell membrane permeability. Moreover, it is considered that the construct including the peptide 8 or 9 was transferred into the cell less easily than when the FAM was used alone because such a construct was large in size.

Thus, it is understood that the amino acid sequences GD and GE do not have the cell membrane permeability but the amino acid sequence including the repeated sequence of these exhibits the cell membrane permeability.

### [Test 2]

Peptides 10 to 12 shown in Table 3 were prepared. A construct including a peptide m (m is a natural number of 10 to 12) shown in Table 3 was defined as a sample m, and samples 10 to 12 were obtained from Eurofins Genomics K.K. In the prepared samples 10 to 12, the α-amino group of the amino acid residue of the N-terminus in the peptides 10 to 12 were acetylated. Then, the cell membrane permeability of the peptides 10 to 12 was evaluated (Examples 10 to 12) in a manner similar to Test 1 described above. In Reference Example 2, the FAM corresponding to the fluorescent dye was added alone in a manner similar to Reference Example 1. However, the sample concentration in the culture medium in the well was 25 µM in Examples 10 to 12 and the FAM concentration in the culture medium in the well was 25 µM in Reference Example 2. The sample (construct) used in each example is shown in Table 4. Table 4 and Fig. 2 showing values of MFI in Examples.

### [Table 3]

**Table 3**

| Peptide No. | Amino acid sequence | SEQ ID No. |
|---|---|---|
| 10 | GDGDGDGDGDGDG | 12 |
| 11 | GEGEGEGEGEGEG | 13 |
| 12 | GDGEGDGEGDGEG | 14 |

### [Table 4]

**Table 4**

| | Structure of construct (additive) | MFI |
|---|---|---|
| Example 10 | Ac-GDGDGDGDGDGDG-FAM | 30.3 |
| Example 11 | Ac-GEGEGEGEGEGEG-FAM | 28 |
| Example 12 | Ac-GDGEGDGEGDGEG-FAM | 28.2 |
| Reference Example 2 | FAM | 13.1 |

As shown in Table 4 and Fig. 2, the value of MFI was drastically higher in Examples 10 to 12 than in Reference Example 2. This indicates that the peptides 10 to 12 have the excellent cell membrane permeability. In addition, since the value of MFI was higher in Examples 10 to 12 than in Examples 1 to 7 in Test 1, it is considered that having the amino acid sequence in which GD or GE is repeated six times can exhibit the excellent cell membrane permeability.

Although the detailed data are not shown, the present inventor's examination has indicated that, in the constructs with the peptides 1 to 7 and 10 to 12, the foreign substance is transferred efficiently from outside the cell into the cytoplasm not only when the foreign substance is the fluorescent dye (for example, FAM) but also when the foreign substance is any of polypeptides, nucleic acids, and drugs.

The specific examples of the art disclosed herein have been described above in detail; however, these are just examples and will not limit the scope of claims. The art described in the scope of claims includes those in which the specific examples given above are variously modified and changed.

### [Industrial Applicability]

The art disclosed herein provides the carrier peptide fragment and the construct including the carrier peptide fragment that can transfer a target foreign substance from outside the eukaryotic cell (in particular, various animal cells typified by human and nonhuman mammalian cells that do not have a cell wall) into the cytoplasm thereof. By utilizing this construct, the target foreign substance can be effectively transferred into the target cell, and biological tissues such as organs and cells to which the foreign substance has been transferred can be obtained. In addition, by utilizing the carrier peptide fragment disclosed herein in techniques of drug delivery, therapeutic agents for various diseases can be provided.

## Claims

1. A synthetic peptide for transferring a target foreign substance from outside a eukaryotic cell into at least a cytoplasm of the cell, the synthetic peptide comprising any of the following amino acid sequences:
(1) an amino acid sequence in which two or more minimum constituent units are bonded continuously in series, the minimum constituent unit being GD (glycine residue-aspartic acid residue) or GE (glycine residue-glutamic acid residue); and
(2) an amino acid sequence in which one to three glycine residues are bonded to a C-terminus of (1) the amino acid sequence.

2. The synthetic peptide according to claim 1, wherein each of (1) the amino acid sequence and (2) the amino acid sequence includes two or more and six or less of the minimum constituent units.

3. A synthetic peptide for transferring a target foreign substance from outside a eukaryotic cell into at least a cytoplasm of the cell, the synthetic peptide comprising any of the following amino acid sequences:
GDGD (SEQ ID No.: 1);
GEGE (SEQ ID No.: 2);
GDGEG (SEQ ID No.: 7);
GEGDG (SEQ ID No.: 8);
GDGDGDG (SEQ ID No.: 9);
GDGDGDGDG (SEQ ID No.: 10);
GDGDGDGDGDG (SEQ ID No.: 11);
GDGDGDGDGDGDG (SEQ ID No.: 12);
GEGEGEGEGEGEG (SEQ ID No.: 13); and
GDGEGDGEGDGEG (SEQ ID No.: 14).

4. A construct for transferring a foreign substance that is manufactured in order to transfer a target foreign substance from outside a eukaryotic cell into at least a cytoplasm of the cell, the construct comprising:
the synthetic peptide according to any one of claims 1 to 3; and
the target foreign substance that is bonded to an N-terminus and/or a C-terminus of the synthetic peptide.

5. The construct according to claim 4, wherein the foreign substance is at least one kind of organic compound selected from the group consisting of a polypeptide, a nucleic acid, a dye, and a drug.

6. The construct according to claim 4, wherein the foreign substance is disposed on the C-terminus of the synthetic peptide.
